**Europäisches Patentamt**

(19) **European Patent Office**

**Office européen des brevets**

(11) Publication number : **0 382 551 B1**

(12) **EUROPEAN PATENT SPECIFICATION**

(45) Date of publication of patent specification :
10.11.93 Bulletin 93/45

(51) Int. Cl.⁵ : **A61K 35/84, A61K 31/715**

(21) Application number : **90301373.8**

(22) Date of filing : **09.02.90**

(54) **Prevention and treatment of herpes virus infections.**

(30) Priority : **10.02.89 JP 32058/89**

(43) Date of publication of application :
**16.08.90 Bulletin 90/33**

(45) Publication of the grant of the patent :
**10.11.93 Bulletin 93/45**

(84) Designated Contracting States :
**DE FR GB IT**

(56) References cited :
**EP-A- 0 292 601**
**US-A- 4 629 627**
**JPN. J. BACTERIOL., vol. 41, no. 3, 1986, page 634; M. TAKEHARA et al.: "Anti herpes virus action of fungal polysaccharides"**

(73) Proprietor : **NIHON CHEMICAL RESEARCH KABUSHIKI KAISHA also known as JCR PHARMACEUTICALS CO., LTD**
**4-20, Mikagehommachi 3-chome**
**Higashinada-ku**
**Kobe Hyogo 658 (JP)**
Proprietor : **NODA SHOKUKIN KOGYO KABUSHIKI KAISHA, also known as NODA SHOKUKIN KOGYO CO., LTD.**
**121, Shimizu**
**Noda-shi, Chiba-ken (JP)**

(72) Inventor : **Koga, Junichi**
**80-5, Karibadai 1-chome, Nishi-ku**
**Kobe, Hyogo 673 (JP)**
Inventor : **Ohashi, Yasuhiro**
**51-11, Iwana 1-chome**
**Noda, Chiba 278 (JP)**
Inventor : **Hiratani, Hajime**
**705-3, Tottori, Hannan-cho**
**Sennan-gun, Osaka 599-02 (JP)**

(74) Representative : **Burford, Anthony Frederick et al**
**W.H. Beck, Greener & Co. 7 Stone Buildings**
**Lincoln's Inn**
**London WC2A 3SZ (GB)**

EP 0 382 551 B1

## Description

This invention relates to inhibitory agents against adsorption of herpes viruses to cells.

It is well known that herpes viruses act as causative agents for various diseases. As an example of herpes viruses, there may be mentioned herpes simplex virus, which is one of the most representatives of the family Herpetoviridae consisting of varicella herpes zoster, cytomegalovirus, EB virus, etc. Herpes simplex virus contains double-stranded DNA with a molecular weight of 85 to 100 x 10$^6$ daltons which is enclosed in an regular icosahedral-formed capsid and an envelope. The virus has been known to have on its envelope six to eight species of glycoproteins with different antigenicities (Longnecker et al.; Proc. Natl. Acad. Sci. USA 84, 4302 (1987)).

This virus can be further classified into two subtypes, herpes simplex virus type 1 and type 2, which are considered to cause mainly stomatitis and genital herpes, respectively, but there is no strict distinction between these two subtypes. The typical diseases, for which this virus is regarded to be responsible, include not only the two described above but also a wide variety of diseases, such as keratitis, menigism, upper and lower respiratory tract infections and facial palsy. The virus, after initial infection, causes hosts to acquire inapparent infection, thereby making almost all human adults positive to its antibody. However, the antibody negativity ratio in adults has risen recently, and the initial infection of herpes simplex virus in expectant and nursing mothers causes extremely severe diseases in fetuses.

Development of various antiviral agents being directed toward this virus has heretofore been under progress, but very few drug substances have been clinically confirmed so far for efficacy; only adenine arabinoside and aciclovir are clinically effective.

Aciclovir as mentioned above, though it is considered to produce enhanced efficacy, shows a high degree of toxicity just as is the case with other nucleic acid antagonists. Under these circumstances, there is strongly demanded a safer and more effective medicine that can demonstrate therapeutic effect through a different mechanism of action.

In addition, there has not been any satisfactory medicine, available so far, that is capable of effectively inhibiting infection with the family Herpetoviridae inclusive of herpes simplex virus, without producing serious side effects to human body. This invention is intended to provide a medicine with a remarkably lowered degree of toxicity, which is quite different from nucleic acid analogs, in order to inhibit the adsorption of herpes viruses to cells and to block the proliferation of such viruses.

In recent years, anti-tumor factors contained in some components of Lentinus edodes have been in clinical use. Such anti-tumor factors, as exemplified by Krestin (Yanagawa et al.; "Gan to Kagakuryohou (Cancer and Chemotherapy), 11, 2155 (1985)) and Lentinan (Suga et al.; Cancer Research, 44, 5162 (1984)), are considered to owe their pharmacological activities to glycoprotein polysaccharide components contained therein. In addition, polysaccharides having sulfuric acid radicals such as dextran sulfate sodium were reported to exhibit activities against AIDS virus (Mitsuya et al., "Science", 248, 646 (1988)).

Furthermore, an aqueous extract of mycelial cultures of Lentinus edodes is called briefly "LEM", and a process for the production of the same is described in Japanese Patent Publication No. Sho 53-10117. LEM has been utilized as an agrochemical effective for the suppression of tobacco mosaic virus, and its therapeutic effect for hepatitis is also suggested (Harada et al.; "Kan-Tan-Sui, 14, No. 2, 327: Sugano et al.; "Cancer Letters", 17, 109 (1982); Suzuki et al.; Igaku no Ayumi (Progress on Medicine) 138 441 (1986)). LEM, when assayed with RNA of tobacco mosaic virus as a substrate, was confirmed to exhibit inhibition of the activity of reverse transcriptase derived from bird myeloma virus. This drug substance, when analyzed for its sugar composition, was found to be specifically characterized by an extremely high content each of arabinose and xylose, indicating that the substance is clearly in contrast with Krestin and Lentinan which both have a sugar composition constituted almost exclusively of glucose.

The use of certain polysaccharide extracts of Lentinus edodes for the treatment of herpes virus has been reported by C. Iizuka (see US-A-4,629,627) and M. Takehara et al (Jpn. J. Bacteriol. 41 (3), 1986, 634).

The present inventors, after continued research on LEM, found that certain fractions of this substance surprisingly possesses an exceedingly strong adsorption inhibitory activity against herpes simplex virus.

The present inventors reacted LEM solutions of different concentrations with dilutions of various viruses at 37°C for 1 to 3 hours and then contacted with animal cells sensitive to these individual viruses. After washing out of the virus dilutions, the presence and number of plaque formations on tissues were observed for 1 to 14 days. LEM showed extremely strong inhibition of plaque formation by herpes viruses, among other viruses. For example, the substance at concentrations of 5 to 10 μg/ml exhibited 50% inhibition against herpes simplex virus on monkey kidney Vero cells, whereas such activity was not detected at all in Krestin and Lentinan. By using this procedure as a guidance, the present inventors developed the fractionation and purification of LEM; an aqueous solution of this substance was subjected to precipitation with an organic solvent, such as methanol

2

and ethanol, and the resultant precipitate was dissolved in water or a buffer, for example phosphate buffer, followed by fractionation with use of ion exchange chromatography and hydrophobic chromatography. The individual fractions thus obtained were further fractionated by use of gel permeation chromatography, with the result that a fraction which exists in electrically neutral range (pH 5 to 8.5) and has a molecular weight corresponding to 10,000 to 1,000,000 daltons was proven to demonstrate strong activity.

From the fact that this fraction, after having its protein removed, showed attenuated activity and that its activity correlated with its molecular weight distribution, protein distribution and sugar distribution, furthermore, it was suggested that the active factor could be composed essentially of a glycoprotein or proteoglycan. Some of the fractions thus obtained, at the concentration of 1μg/ml demonstrated 50% inhibition against herpes simplex virus (HSV) type 1, while showing similar activity against HSV type 2. Dextran sulfate sodium, when assayed in this system, also exhibited the same inhibitory activity but required the minimum concentration of 5 μg/ml to achieve 50% inhibition against HSV type 1, with higher concentration being needed for 50% inhibition against HSV type 2. LEM, when its acute toxicity through oral administration was determined in rats and mice, showed a $LD_{50}$ value of 15 g/kg, being proven to be extremely low in toxicity. In the subacute toxicity test, the substance produced no dose-dependent change, except some animals were observed to develop loose faeces which was assumed to be caused by dyspepsia.

This invention is based on the above described finding, and is concerned with a treatment agent for diseases caused by herpes viruses or an inhibitory agent against adsorption of such herpes viruses which contains a fraction of an aqueous extract of a mycelial culture of Lentinus edodes having a molecular weight corresponding to 10,000 to 1,000,000 daltons.

Cultivation of mycelia of Lentinus edodes has been carried out widely for the purpose of collecting fruit bodies, but in practicing this invention, it is preferred to extract the resultant mycelial culture with warm water in the stage just prior to the formation of fruit bodies.

As the aqueous extract in this invention, there may be used an extract (LEM, on the market) obtainable by conducting the cultivation and extraction in accordance with the procedure described in Japanese Patent Publication Sho 53-10117 (1978), whereupon a strain of Lentinus edodes is cultured in a culture medium composed of bagasse and defatted rice bran. The fraction having a molecular weight corresponding to 10,000 to 1,000,000 in the aqueous extract can be produced by subjecting the aqueous extract to gel permeation chromatography.

The fraction as described above can be administered parenterally or orally in order to inhibit the adsorption of herpes viruses to cells.

In order to treat skin symptoms brought about by the attack of herpes viruses or to inhibit the adsorption of the viruses, the fraction according to this invention can desirably be used in the form of preparations for external use being suited for local application to, for example, the skin or mucosa attacked or possibly attacked by the viruses. Locations of such skin and mucosa include openings of human body, such as the oral cavity, throat, nostril, eyelids, anus, rectum, urethra and vagina as well as injured portions and surroundings thereof.

The treatment or therapeutic agent or inhibitor of viral adsorption according to this invention, as processed in the form of preparations for external use, can be administered to a subject in such dosage forms as a suppository, jelly, cream, cataplasma, ointment, plaster, injunction, liquids, spray, aerosol and powder for external use, as the case may be. Such preparations for external use can be prepared by the procedures known per se.

In order to prevent decomposition of the active ingredient with water during storage, for example, the fraction is desirably freeze-dried, followed by addition of water to the lyophilizate on the occasion of use.

The fraction of this invention may be administered orally in the dosage forms, such as a powder, granule, pill and tablet, if desired. Such pharmaceutical preparations can be produced by conventional methods.

In addition, the fraction can also be utilized for injection in the form of aqueous solution.

The fraction of this invention is preferably used in the proportions of 1 to 5% as LEM in such preparations for external use, while it is desirably employed at a single dose of 10 to 10,000 mg for oral administration. The fraction can be used in smaller doses than LEM, for example one third to one fifth of the LEM dose.

For the purpose of injection, the fraction can be utilized at a single dose in the range of 1 to 1,000 mg. Injectable solutions are desirably administered by subcutaneous injection.

In the present invention, the pharmaceutical preparations containing the fraction of the aqueous extract of mycelial culture of Lentinus edodes act to inhibit the bonding of herpes viruses to target cells to thereby block the infection with said viruses, onset of diseases caused by the viruses or spread of the affected lesions.

According to this invention, there can be provided medicaments with an enhanced degree of safety being capable of inhibiting the adsorption of herpes viruses to cells.

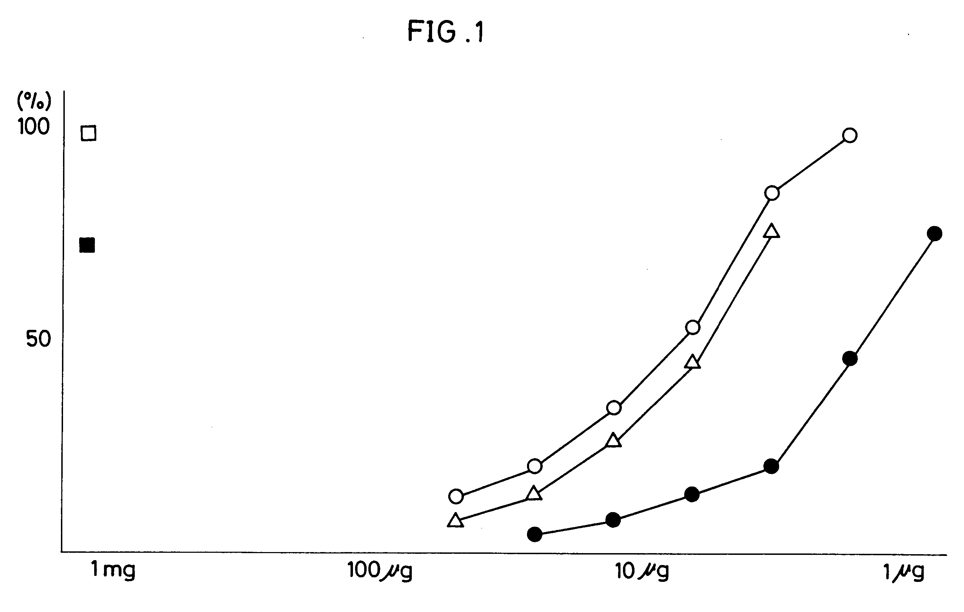

Referring to the drawing, Fig. 1 is a summarized representation of adsorption-inhibitory activities as assayed in Example 3, where the percentages of numbers of plaques from cells treated with diluted solutions of individual test samples divided by numbers of plaques from non-treated cells are plotted as ordinate and concentrations of test samples as abscissa; the symbols, □, ■, Δ, ○ and ● designate Lentinan, Krestin, dextran suflate sodium, LEM and Fraction 4, respectively.

LEM, which was used in the examples described below, is prepared and marketed by Noda Shokukin Kogyo K.K. located at Noda city, Chiba, Japan, and shows physico-chemical properties as given in the following:

Name:

Extract of mycelial culture of Lentinus edodes (LEM)
Constituents:

| | |
|---|---|
| Sugar | ; 30 to 38% |
| Protein | ; 10 to 12% |
| Ash | ; 16 to 18% |
| Water | ; 6 to 8% |

Sugar composition:

| | |
|---|---|
| Arabinose | ; 35.6 to 39.0% |
| Xylose | ; 28.8 to 30.8% |
| Glucose | ; 16.3 to 20.0% |
| Mannose | ; 7.6 to 8.4% |
| Galactose | ; 4.9 to 6.3% |
| Fucose+Rhamnose | ; 0.3 to 1.7% |

Appearance:
Brownish to dark brown granule or powder, bitter taste.
Solubilities:
The substance is very slightly soluble in water and almost insoluble in ethanol and ether.
Hygroscopicity:
The substance is hygroscopic.

Example 1

With a specific view to investigation into typical physical properties of the active factor contained in LEM, purification was carried out. 120 g of LEM was dissolved in 1 liter of distilled water, and after insoluble matter was removed by centrifugation, the supernatant liquid was fractionated by means of hydrophobic chromatography on Phenyl Sepharose™ (Pharmacia Co., Sweden) and gel permeation chromatography on Sephacryl™ S-300 (Pharmacia Co., Sweden).

The fractionation procedure is shown in the scheme of Table 1, while the sugar compositions of the resultant fractions are tabulated in Table 2.

Table 1: Fractionation of LEM

120 g of LEM

├— 4 liters of distilled water
├— Addition of ethanol (37.5%)
├— Centrifugation/discard of precipitate
├— Addition of ethanol (50%)
├— Centrifugation/recovery of precipitate
├— Dissolution into 250 ml of 1M aq. ammonium sulfate
├— Adsorption on Phenyl Sepharose$^{Tm}$

| Elution with phosphate buffer | | | Elution with ethylene glycol | | | |
|---|---|---|---|---|---|---|
| Gel permeation | | | Gel permeation | | | |
| Fr*.5 | Fr. 6 | Fr. 7 | Fr. 1 | Fr. 2 | Fr. 3 | Fr. 4 |
| $10^6$ | $10^5$-$10^6$ | $10^4$-$10^5$ | $2 \times 10^6$ | $5 \times 10^5$-$2 \times 10^6$ | $10^5$-$5 \times 10^5$ | $10^4$-$10^5$ |

Note, *; stands for fraction. Figures designate molecular weight ranges

Fr. 4 exhibited outstanding activity against herpes viruses (see Example 2) but the remaining fractions also exhibited activity in the following order:

Fr. 4 > Fr. 3 > Fr. 2 > Fr. 1 > Fr. 5 > Fr. 6 > Fr. 7.

Table 2: Sugar composition of each fraction

| | Glucose (%) | Xylose (%) | Galactose (%) | Arabinose (%) | Mannose (%) |
|---|---|---|---|---|---|
| Fraction 1 | 77.9 | 12.6 | 4.2 | 5.3 | - |
| Fraction 2 | 30.3 | 45.7 | 5.7 | 18.3 | - |
| Fraction 3 | 30.9 | 48.1 | 4.3 | 16.7 | - |
| Fraction 4 | 25.5 | 48.5 | 5.6 | 17.3 | 3.1 |
| Fraction 5 | 81.0 | 8.0 | 2.7 | 6.0 | 2.3 |
| Fraction 6 | 65.2 | 2.3 | - | 32.2 | 5.2 |
| Fraction 7 | 49.3 | 43.7 | 7.0 | - | - |
| Krestin | 72.2 | 4.0 | 4.5 | - | 19.3 |
| Lentinan | 72.6 | - | 5.9 | 12.2 | 9.3 |

### Example 2

LEM was assayed for inhibitory activity against adsorption of viruses. Monkey kidney Vero cells were grown on a 24-holes plate (Nunc Co., Denmark) for tissue cultivation. Strain F of herpes simplex virus (distributed by Prof. R. J. Whitley of Clinical Virology Lab., University of Alabama) was diluted with a culture medium composed of Eagle MEM (Nissui Pharmaceuticals Co, Japan) containing 1% of serum (Dai-Nippon Pharmaceuticals Co., Japan) in such a manner as it contains 50 to 100 plaque forming units in 0.1 ml. Stepwise diluted solutions of LEM or its fraction and stepwise diluted solutions of a control drug were mixed with the diluted solution of the virus in equal portions, individually, to undergo reaction at 37°C for 1 to 2 hours.

After washing cells in the plate with phosphate-buffer isotonic saline of Dulbecco's formulation (PBS, Nissui Pharm. Co., Japan), 0.1 ml portions of the reaction solution were poured into individual holes and contacted with cells for 1 hour. The cells were washed with PBS and overlaid for solidification with the above-mentioned culture medium containing 0.2% of agar, followed by cultivation in a $CO_2$ incubator for 2 days. In counting a number of plaques, the cells in individual holes were fixed with each 1 ml of 10% formalin (Wako Pure Chemicals Co., Japan) and dyed with each 0.5 ml of 0.03% Methylene Blue (Sigma Co., U.S.A.).

The results are shown in Fig. 1, where the percentages of numbers of plaques from the cells treated with each diluted solution of LEM divided by the ones from the cells not treated with LEM is plotted as ordinate and the concentrations of test samples as abscissa. In this figure, The symbols, □, ■, Δ, ○, and ● designate Lentinan, Krestin, dextran sulfate sodium, LEM and Fraction 4, respectively. The results indicate that LEM at the concentration of 5 μg/ml and Fraction 4 at 1 μg/ml (the concentrations as converted to sugar one) are capable of 50% inhibiting the plaque formation on the treated cells against the control. Fraction 4 exhibited inhibitory activity 500-fold or more as potent as Krestin and Lentinan as assayed together, while it developed the activity at lowered concentrations as compared with dextran sulfate sodium.

### Example 3

Dissolved in 0.025M phosphate buffer (pH 6.8) containing 0.9% (W/V) of sodium chloride was 5,000 ml (50 μg/ml) of Fraction 4 as obtained in Example 1, and the solution was sterile-filtered by use of Millipore™ filter and distributed in 2 ml portions into ampoules, followed by lyophilization to produce preparations for injection.

### Example 4

In 10 ml of isotonic saline was dissolved 1 g of Fraction 4, and hydrophilic ointment was added little by little to the solution, while kneading, to produce 100 g of ointment.

## Claims

1. A fraction of an aqueous extract of a mycelial culture of <u>Lentinus</u> <u>Edodes</u>, said fraction corresponding to molecular weights of 10,000 to 1,000,000 daltons and having a sugar composition comprised of arabinose, xylose, glucose, mannose, and galactose.

2. A fraction as claimed in Claim 1, which corresponds to molecular weights of 10,000 to 100,000.

3. A fraction as claimed in Claim 1 or Claim 2 obtainable by precipitation of aqueous LEM with ethanol, ion-exchange and hydrophobic chromatography and gel permeation chromatography.

4. A fraction as claimed in Claim 3, wherein said aqueous extract has been obtained by cultivating a strain of <u>Lentinus</u> <u>Edodes</u> in a culture medium composed of bagasse and rice bran, followed by extracting the resultant culture, prior to the formation of fruit bodies, with warm water.

5. A pharmaceutical composition for the prevention or treatment of herpes virus infections comprising a fraction as claimed in Claim 1.

6. A pharmaceutical composition as claimed in Claim 5, wherein said fraction is as defined in any one of Claims 2 to 4.

7. The use in preparing a medicament for the prevention or treatment of herpes virus infections of a fraction as claimed in Claim 1.

8. A use as claimed in Claim 7, wherein said fraction is as defined in any one of Claims 2 to 4.

9. A use as claimed in Claim 7 or Claim 8, wherein said medicament is in a form suited for topical application to skin or mucosa portions of the human body or vicinity thereof.

10. A use as claimed in Claim 9, wherein said medicament contains 0.1 to 5 weight % (converted into LEM) of said extract or fraction.

11. A use as claimed in Claim 7 or Claim 8, wherein said medicament is in a form for oral administration and each dosage unit contains 10 to 10,000 mg (converted into LEM) of said extract or fraction.

12. A use as claimed in Claim 7 or Claim 8, wherein said medicament is in the form of an injection containing 1 to 1,000 mg per dose of said fraction.

13. A fraction as claimed in Claim 1 for use in the prevention or treatment of herpes virus infections.

14. A fraction as claimed in any one of Claims 2 to 4 for use in the prevention or treatment of herpes virus infections.


**Patentansprüche**

1. Fraktion von einem wässrigen Extrakt von einer Myzelialkultur von Lentinus Edodes, die Molekulargewichten von 10.000 bis 1.000.000 Dalton entspricht und eine Arabinose, Xylose, Glukose, Mannose und Galaktose enthaltende Zuckerzusammensetzung aufweist.

2. Fraktion nach Anspruch 1, die Molekulargewichten von 10.000 bis 100.000 entspricht.

3. Fraktion nach Anspruch 1 oder 2, erhältlich durch Präzipitation von wässrigem LEM mit Ethanol, Ionaustausch- und hydrophobe Chromatographie und Gelpermations-Chromatographie.

4. Fraktion nach Anspruch 3, worin der wässrige Extrakt durch Kultivierung eines Stammes von Lentinus Edodes in einem aus Bagasse und Reiskleie zusammengesetzten Kulturmedium, gefolgt von einer Extraktion der erhaltenen Kultur vor Bildung der Fruchtkörper mit warmem Wasser, erhalten wurde.

5. Pharmazeutische Zusammensetzung zur Prävention oder Behandlung von Herpesvirusinfektionen, enthaltend eine Fraktion nach Anspruch 1.

6. Pharmazeutische Zusammensetzung nach Anspruch 5, in der die Fraktion einer der Definitionen der Ansprüche 2 bis 4 entspricht.

7. Verwendung einer Fraktion nach Anspruch 1 zur Herstellung eines Medikamentes zur Prävention oder Behandlung von Herpesvirusinfektionen.

8. Verwendung nach Anspruch 7, worin die Fraktion der Definition in einem der Ansprüche 2 bis 4 entspricht.

9. Verwendung nach Anspruch 7 oder 8, worin das Medikament in einer für die topische Verabreichung auf Haut- oder Schleimhautbereiche des menschlichen Körpers oder umgebende Bereiche geeigneter Form vorliegt.

10. Verwendung nach Anspruch 9, worin das Medikament 0,1 bis 5 Gew.-% (überführt in LEM) des Extraktes oder der Fraktion enthält.

11. Verwendung nach Anspruch 7 oder 8, worin das Medikament in Form einer für die orale Verabreichung geeigneten Form vorliegt und jede Dosiereinheit 10 bis 10.000 mg (überführt in LEM) des Extraktes oder der Fraktion enthält.

12. Verwendung nach Anspruch 7 oder 8, worin das Medikament in einer Injektionsform enthaltend 1 bis 1.000 mg pro Dosis der Fraktion vorliegt.

**13.** Fraktion nach Anspruch 1 zur Verwendung in der Prävention oder der Behandlung von Herpesvirusinfektion.

**14.** Fraktion nach einem der Ansprüche 2 bis 4 zur Verwendung in der Prävention oder Behandlung von Herpesvirusinfektionen.


**Revendications**

**1.** Fraction d'un extrait aqueux d'une culture mycélienne de *Lentinus edodes* (LEW), ladite fraction correspondant à des poids moléculaires de 10 0000 à 1 000 000 daltons et ayant une composition en sucres comprenant de l'arabinose, du xylose, du glucose, du mannose et du galactose.

**2.** Fraction selon la revendication 1, qui correspond à des poids moléculaires de 10 000 à 100 000.

**3.** Fraction selon la revendication 1 ou 2, qui peut être obtenue par précipitation par l'éthanol de LEM aqueux, chromatographie hydrophobe et d'échange d'ions, et chromatographie de perméation sur gel.

**4.** Fraction selon la revendication 3, dans laquelle ledit extrait aqueux a été obtenu par culture d'une souche de *Lentinus edodes* dans un milieu de culture composé de bagasse et de son de riz, suivie d'extraction de la culture résultante, avant la formation de corps fructifères, avec de l'eau chaude.

**5.** Composition pharmaceutique pour la prévention ou le traitement d'infections liées aux virus de l'herpès, comprenant une fraction selon la revendication 1.

**6.** Composition pharmaceutique selon la revendication 5, dans laquelle ladite fraction est telle que définie dans les revendications 2 à 4.

**7.** Utilisation d'une fraction selon la revendication 1 dans la préparation d'un médicament pour la prévention ou le traitement d'infections liées aux virus de l'herpès.

**8.** Utilisation selon la revendication 7, dans laquelle ladite fraction est telle que définie dans l'une quelconque des revendications 2 à 4.

**9.** Utilisation selon la revendication 7 ou la revendication 8, dans laquelle ledit médicament est sous une forme appropriée pour l'application locale à des portions de peau ou de muqueuse du corps humain ou à leur voisinage.

**10.** Utilisation selon la revendication 9, dans laquelle ledit médicament contient 0,1 à 5% en poids (transformé en LEM) dudit extrait ou fraction.

**11.** Utilisation selon la revendication 7 ou 8, dans laquelle ledit médicament est sous une forme pour administration orale et chaque unité posologique contient 10 à 10 000 mg (transformé en LEM) dudit extrait ou fraction.

**12.** Utilisation selon la revendication 7 ou 8, dans laquelle ledit médicament est sous la forme d'un produit pour injection contenant 1 à 1 000 mg de ladite fraction par dose.

**13.** Fraction selon la revendication 1, pour l'utilisation dans la prévention ou le traitement des infections liées aux virus de l'herpès.

**14.** Fraction selon l'une quelconque des revendications 2 à 4, pour l'utilisation dans la prévention ou le traitement des infections liées aux virus de l'herpès.

FIG .1

EP 0 382 551 B1